# EUROPEAN PATENT APPLICATION

(11) **EP 4 428 873 A1**
(43) Date of publication of application: **11.09.2024**
(21) Application number: 24162036.8
(22) Date of filing: 07.03.2024
(51) Int. Cl.: G16H 50/20, G16H 50/70, G16H 10/00, G16H 30/40, A61B 5/055

(54) **FOREIGN OBJECT DETECTION FOR MAGNETIC EXPOSURE**

(30) Priority: 08.03.2023 US 202318118992
(71) Applicant: Optum, Inc., Minnetonka, Minnesota 55343 (US)
(72) Inventor: VIAL, Paul Alain, Vancouver, V5V1A5 (CA); DUBOIS, David, Mirabel, J7J0K9 (CA); DANESHVAR, Sara, Port Moody, VH0L3 (CA); BASHAM, Rachel H., Huntsville, 35811 (US)
(74) Representative: Zimmermann & Partner Patentanwälte mbB

(57) **Abstract**

A system and method for screening for implants and/or foreign objects prior to undergoing magnetic exposure (e.g., an MRI) utilizing artificial intelligence (Al) analysis of medical imaging information and/or medical records information.

## Description

### BACKGROUND

Magnetic exposure to the body, such as that occurs during magnetic resonance imaging (MRI), requires knowledge of any implanted or foreign object within the body. For example, during an MRI scan, serious injury may occur as the result of movement or dislodgement of a metallic foreign object in a patient's body due to the powerful magnetic field. Additionally, excessive heating may occur because of a foreign object.

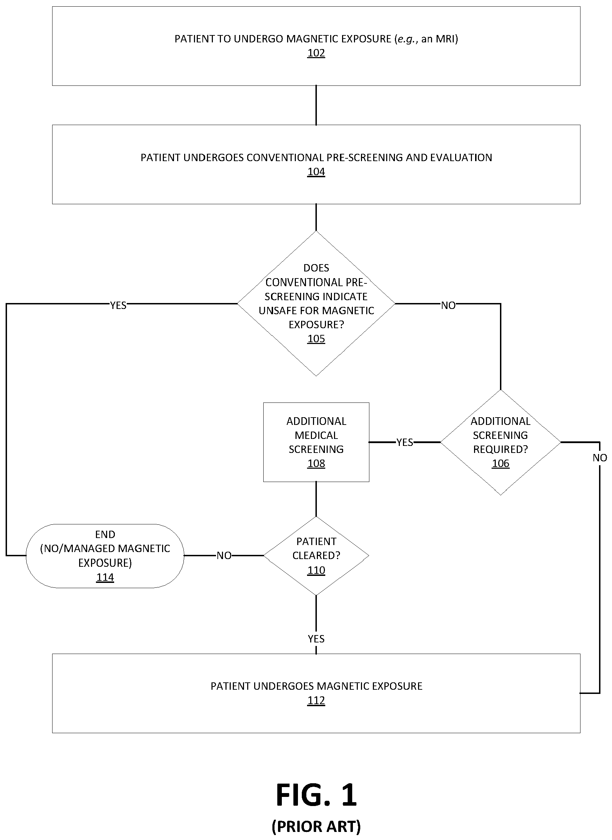

Conventionally, as shown in FIG. 1, a patient that is to undergo magnetic exposure (such as an MRI) 102, is screened and evaluated 104 prior to the magnetic exposure (e.g., an MRI exam), as any undetected foreign objects result in a serious risk to patient safety. Conventional screening and evaluation 104 comprises the patient completing a mandatory screening form. However, a patient may misunderstand what should be considered a foreign object, or they may forget about a foreign object in their body. In some instances, the patient may have a foreign object in their body that they are unaware of (e.g., metal slivers). If a patient is unconscious or mentally unable to complete the form, the individual who completes the form for them may not be aware of foreign objects in the patient's body. Completion of the screening form may delay image acquisition, which may be problematic for emergency patients. Based on the patient's screening and evaluation, a determination is made at 105 whether the pre-screening and evaluation indicate it is unsafe for the patient to undergo the magnetic exposure. If, at 105, it is determined that it is not unsafe for the patient to undergo the magnetic exposure, then at 106 it is determined whether additional screening is required. Additional screening may be needed if the patient is unconscious and there is no one familiar with the patient's medical history. If it is determined at 106 that additional screening is needed, then at 108 additional medical screening (e.g., x-rays, CT, metal detection, etc.) may be performed to check for implants and/or foreign objects. Based on the results of the additional medical screening 108, it is determined at 110 whether the patient is cleared for the magnetic exposure. If at 110, the patient is cleared, then at 112 the patient undergoes the magnetic exposure (e.g., MRI). If the patient is not cleared at 110 (e.g., the additional medical screening 108 shows an implanted or foreign object in the patient), then the process goes to 114, where the magnetic exposure is not performed or the magnetic exposure is managed such that the implanted or foreign object is not exposed to the magnetic fields. Returning to 105, if it is determined that it is unsafe for the patient to undergo magnetic exposure based on the conventional pre-screening at 104 (e.g. the patient indicates that they have an implant or foreign object in their body that is not safe for an MRI), then the process goes to 114 and ends or the magnetic exposure is managed such that the implanted or foreign object is not exposed to the magnetic fields. If, at 105, it is determined that it is not unsafe for the patient to undergo magnetic exposure based on the conventional pre-screening at 104, and it is determined that additional screening is not needed at 106, then the process goes to 112 where the patient undergoes the magnetic exposure. These conventional solutions are dependent on the patient's memory and/or as-needed additional medical screening.

These current solutions may have catastrophic results if they are not correct and can be expensive (questionnaires, additional screening, etc.) and/or time-consuming. Correctly and accurately determining the presence or absence of an implant or foreign object is a challenge using conventional processes and technology. Patient risk can be high and lead to serious injury/death. For example, a death has been reported to the FDA about a patient having an MRI performed and the patient started complaining about a headache. 24-hours later the patient passed away. An ensuing autopsy showed that an aneurysm clip placed in 1978 had been moved by the MRI's magnetic field. Later it was determined to be a ferromagnetic clip, which are unsafe for MRI. There is also a risk of burns from when the implant is removed but the leads for that implant are still left in the body. It is imperative that the MRI technologist is made aware of the leads being left in the body. For example, there is a documented case where the patients experienced permanent neurological injury after undergoing an MRI lumbar exam. This was caused by the leads from a deep brain stimulation device that is used in patients with Parkinson's Disease.

Therefore, systems and methods are desired that overcome challenges in the art, some of which are described above. In particular, an improved system and method of determining the presence of absence of implants and/or foreign objects in a patient's body prior to exposure to a strong magnetic field such as an MRI is desired. More specifically, a patient screening process that uses artificial intelligence (Al) or machine learning (ML)-enabled engine for identifying implants and/or foreign objects in the patient's body from medical imaging information and/or medical records is desired.

### SUMMARY

Described and disclosed herein is a tool comprised of a system, method and computer-program product that improves the screening and determination of implants and/or foreign objects in the body that could be affected by magnetic exposure such as an MRI.

As described herein, a trained AI/ML engine evaluates the pixel data in the patient's prior imaging studies (X-ray, CT, MRI, etc.) to detect the presence of implants and/or foreign objects. If such an object is detected, a warning is generated for use in magnetic exposure (e.g., MRI) pre-exam screening. This warning may be displayed in a picture archiving and communication system (PACS) worklist or sent through an interface for display with the patients records in an external health information system (e.g., one that is used for ordering radiology procedures). Such PACS and radiology management systems are available from, for example, Change Healthcare Inc. (Nashville, Tennessee), among others.

In some instances, a trained natural language processing (NLP) engine may be used to analyze electronic medical records (EMR), clinical reports, emergency department notes, clinical notes, and the like to identify patients with implanted devices and/or known embedded foreign objects.

In use, technologists complete their normal pre-magnetic exposure screening process and review any system-generated foreign object warnings provided by the AI/ML and/or NPL engines described herein.

In some instances, the AI/ML and/or NPL engines can be trained to provide an indication of the MR Conditional categorization of a detected implant and/or foreign object. The terminology of MR Conditional is recognized by the American Society for Testing and Materials (ASTM) international and used by the Food and Drug Administration (FDA). The term "MR Conditional" means that the implant and/or foreign object has to be scanned under certain conditions within the MRI environment. Parameters that are included in the environment are the following, static magnetic field strength, spatial gradient, dB/dt time varying magnetic fields, radio frequency fields, and specific absorption rate (SAR). Additional parameters including, for example, leads for a neurostimulator system, may be required.

In one aspect, a system for screening for implants and/or foreign objects prior to undergoing magnetic exposure (e.g., an MRI) is disclosed. One embodiment of the system comprises at least one computing device. The at least one computing device comprises an artificial intelligence (Al) trained module that has been trained using historical medical information. The system further comprises a memory storing computer-readable instructions that when executed by the at least one computing device cause the at least one computing device to: receive, by the Al trained module, medical information about a patient; analyze, by the Al trained module, the received medical information; and provide, by the Al trained module, information related to performing the magnetic exposure of the patient based on the analysis of the received medical information.

In some instances of the system, the medical information about the patient comprises medical imaging information. In such instances, providing the information related to performing the magnetic screening on the patient based on the analysis of the received medical information comprises the trained Al module analyzing the medical imaging information and indicating a presence or an absence of an implant or a foreign object in the patient's body with a confidence level or probability, and/or indicating an MR Conditional categorization for the implant or the foreign object in the patient's body. In some instances, the information related to performing the magnetic exposure of the patient based on the analysis of the received medical information provided by the trained Al module may be verified by a technologist.

In some instances of the system, the medical information about the patient comprises medical records information. In such instances, providing the information related to performing the magnetic exposure of the patient based on the analysis of the received medical information comprises the trained Al module analyzing the medical records information and indicating a presence or an absence of an implant or a foreign object in the patient's body with a confidence level or probability, and/or indicating an MR Conditional categorization for the implant or the foreign object in the patient's body. In some instances, the information related to performing the magnetic exposure of the patient based on the analysis of the received medical information provided by the trained Al module may be verified by a technologist.

In some instances of the system, the medical information about the patient comprises medical imaging information and medical records information. In such instances, providing the information related to performing the magnetic screening on the patient based on the analysis of the received medical information comprises the trained Al module analyzing the medical records information and the medical imaging information and indicating a presence or an absence of an implant or a foreign object in the patient's body with a confidence level or probability, and/or indicating an MR Conditional categorization for the implant or the foreign object in the patient's body. In some instances, the information related to performing the magnetic exposure of the patient based on the analysis of the received medical information provided by the trained Al module may be verified by a technologist.

In another aspect, a method for screening for implants and/or foreign objects prior to undergoing magnetic exposure (e.g., an MRI). In one embodiment, the method comprises training an artificial-intelligence (Al) module using historical medical records; receiving, by the artificial-intelligence (Al) trained module, medical information about a patient; analyzing, by the Al trained module, the received medical information; and providing, by the Al trained module, information related to performing the magnetic exposure of the patient based on the analysis of the received medical information.

In some instances of the method, the medical information about the patient comprises medical imaging information. In such instances, providing the information related to performing the magnetic exposure of the patient based on the analysis of the received medical information comprises the trained Al module analyzing the medical imaging information and indicating a presence or an absence of an implant or a foreign object in the patient's body with a confidence level or probability, and/or indicating an MR Conditional categorization for the implant or the foreign object in the patient's body. In some instances, the information related to performing the magnetic exposure of the patient based on the analysis of the received medical information provided by the trained Al module is verified by a technologist.

In some instances of the method, the medical information about the patient comprises medical records information. In such instances, providing the information related to performing the magnetic exposure of the patient based on the analysis of the received medical information comprises the trained Al module analyzing the medical records information and indicating a presence or an absence of an implant or a foreign object in the patient's body with a confidence level or probability, and/or indicating an MR Conditional categorization for the implant or the foreign object in the patient's body. In some instances, the information related to performing the magnetic exposure of the patient based on the analysis of the received medical information provided by the trained Al module is verified by a technologist.

In some instances of the method, the medical information about the patient comprises medical imaging information and medical records information. In such instances, providing the information related to performing the magnetic screening on the patient based on the analysis of the received medical information comprises the trained Al module analyzing the medical records information and the medical imaging information and indicating a presence or an absence of an implant or a foreign object in the patient's body with a confidence level or probability, and/or indicating an MR Conditional categorization for the implant or the foreign object in the patient's body. In some instances, the information related to performing the magnetic exposure of the patient based on the analysis of the received medical information provided by the trained Al module is verified by a technologist.

Other systems, methods, features and/or advantages will be or may become apparent to one with skill in the art upon examination of the following drawings and detailed description. It is intended that all such additional systems, methods, features and/or advantages be included within this description and be protected by the accompanying claims.

### BRIEF DESCRIPTION OF THE DRAWINGS

The components in the drawings are not necessarily to scale relative to each other. Like reference numerals designate corresponding parts throughout the several views.
FIG. 1 is an illustration of a conventional method of screening for implants and/or foreign objects in a patient's body prior to undergoing magnetic exposure (e.g., an MRI);

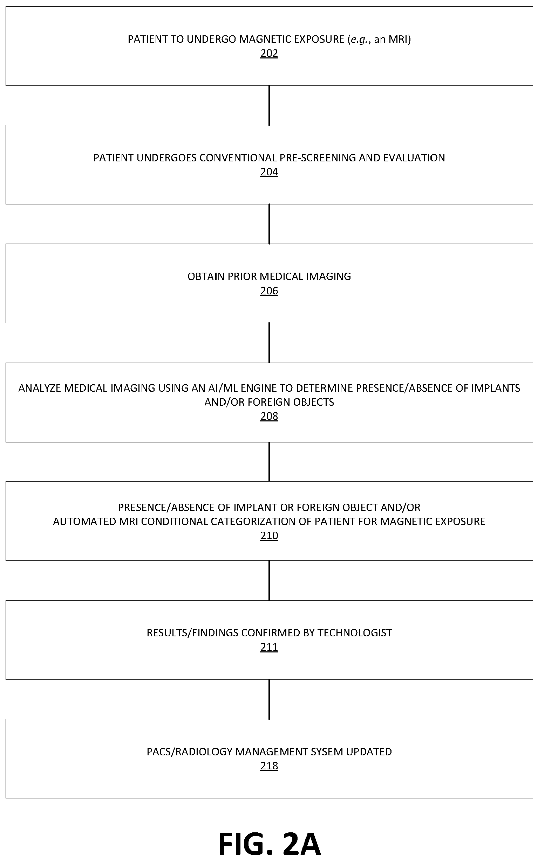

FIG. 2A is an illustration of an improved method of screening for implants and/or foreign objects in a patient's body prior to undergoing magnetic exposure (e.g., an MRI);

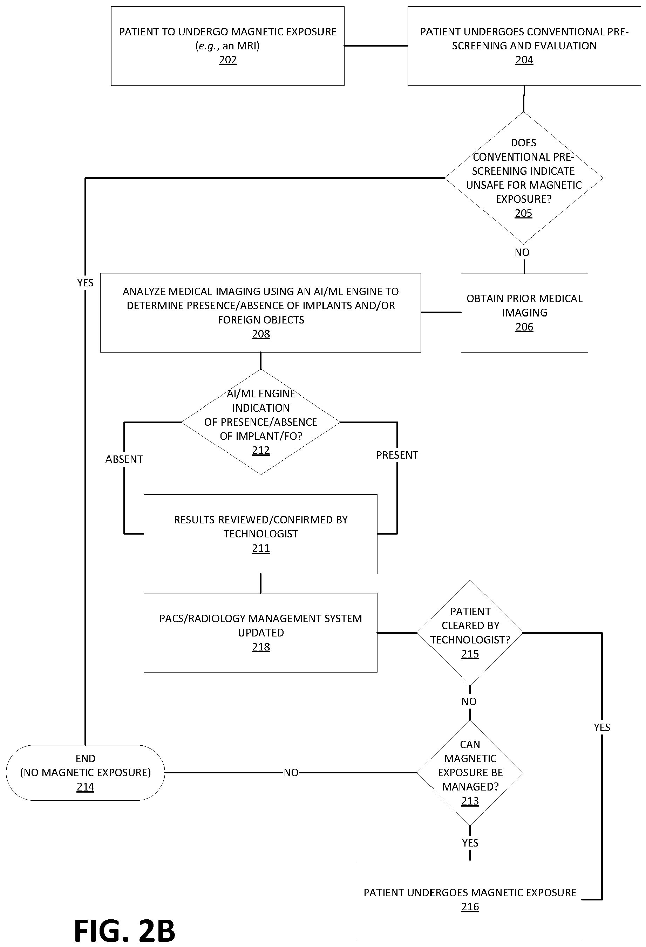

FIG. 2B is an illustration of an improved method of screening for implants and/or foreign objects in a patient's body prior to undergoing magnetic exposure (e.g., an MRI), including performing/not performing the magnetic exposure or managing magnetic exposure of the implant and/or foreign object based on the analysis;

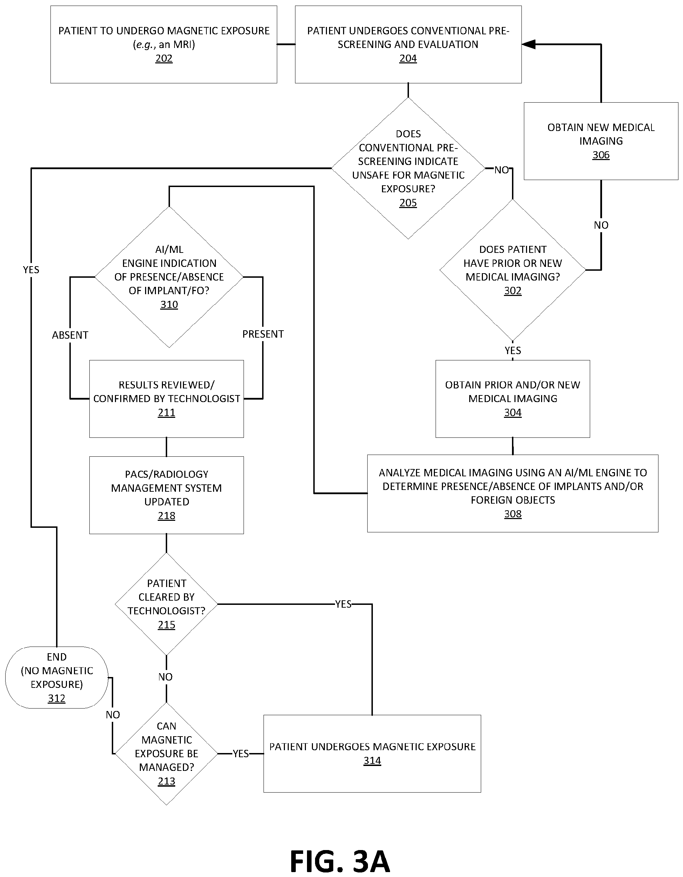

FIG. 3A is an illustration of an improved method of screening for implants and/or foreign objects in a patient's body prior to undergoing magnetic exposure (e.g., an MRI), including determining the availability of any prior medical imaging and/or performing new medical imaging, and performing/not performing the magnetic exposure or managing magnetic exposure of the implant and/or foreign object based on the analysis;

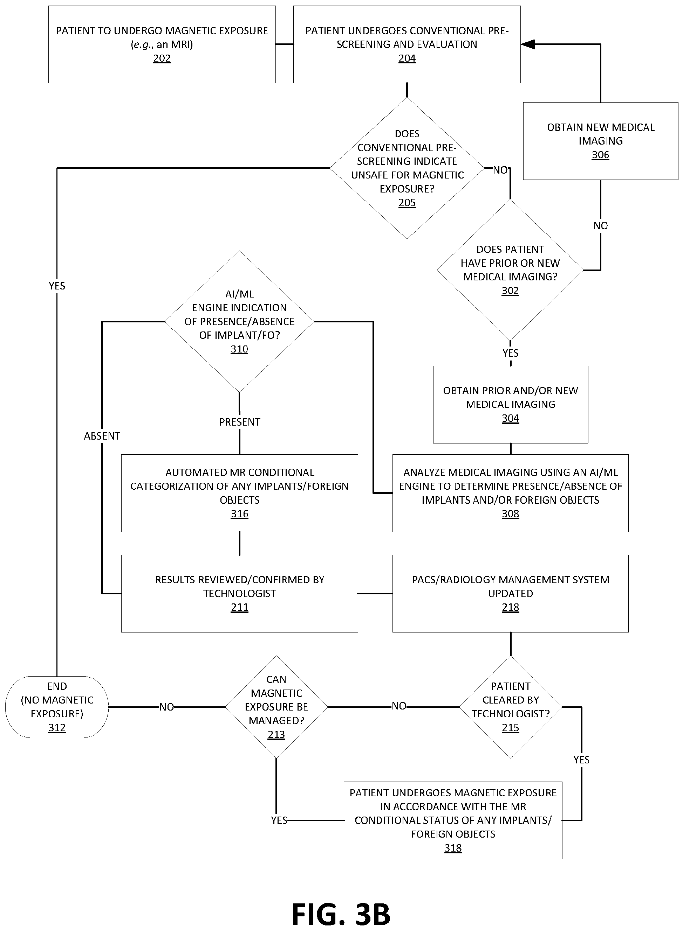

FIG. 3B is an illustration of an improved method of screening for implants and/or foreign objects in a patient's body prior to undergoing magnetic exposure (e.g., an MRI), including determining the availability of any prior medical imaging and/or performing new medical imaging, and providing automated MR Conditional categorization for the patient of any detected implants and/or foreign objects based on the analysis;

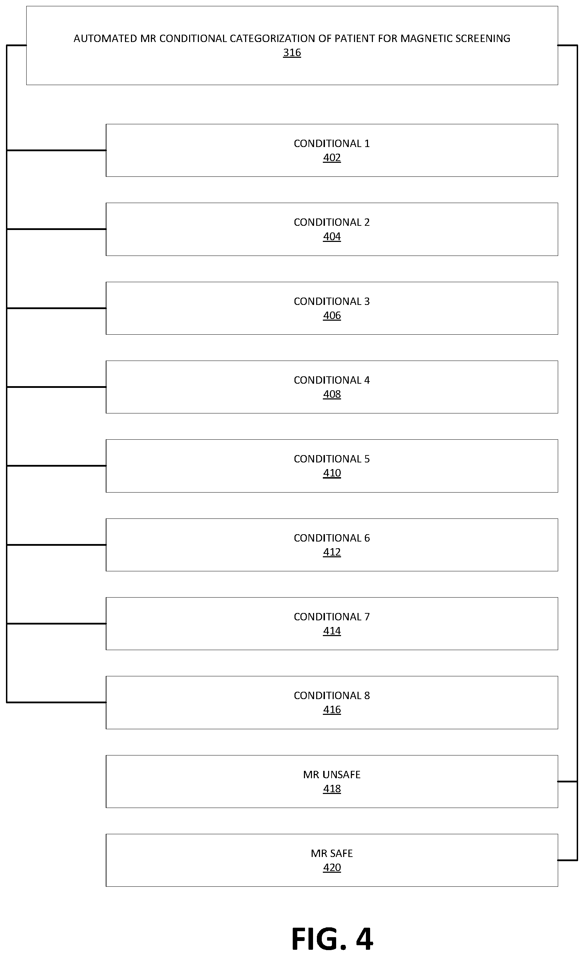

FIG. 4 is an illustration of a conventional set of MR Conditional ratings that can be applied to a patient based on analyzed medical imaging and/or medical records information;

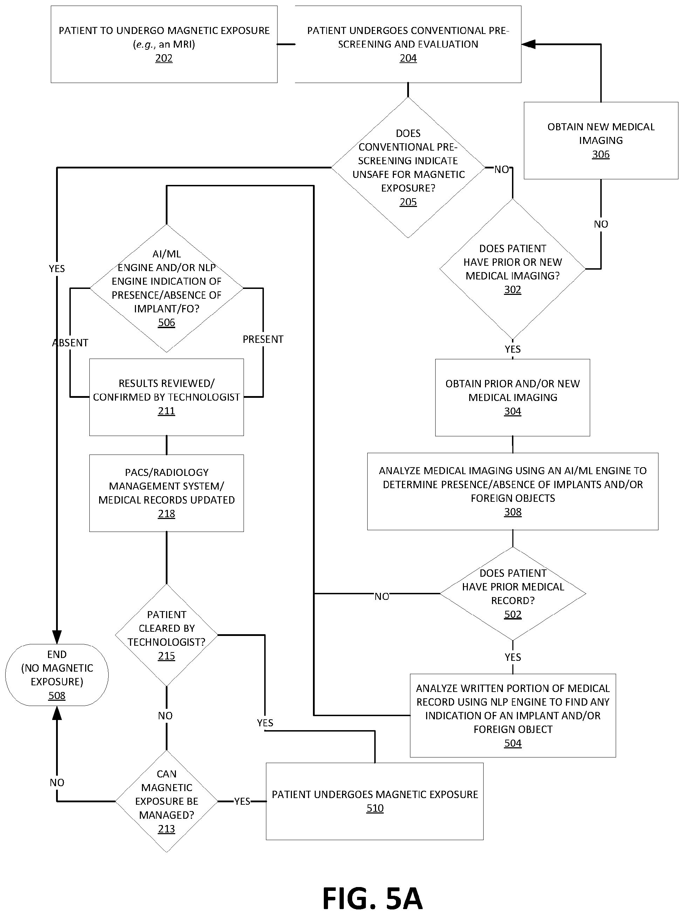

FIG. 5A is an illustration of an improved method of screening for implants and/or foreign objects in a patient's body prior to undergoing magnetic exposure (e.g., an MRI), including determining the availability of any prior medical imaging and/or performing new medical imaging, further including analyzing any textual medical records using a NLP engine, and performing/not performing the magnetic exposure or managing magnetic exposure of the implant and/or foreign object based on the analysis;

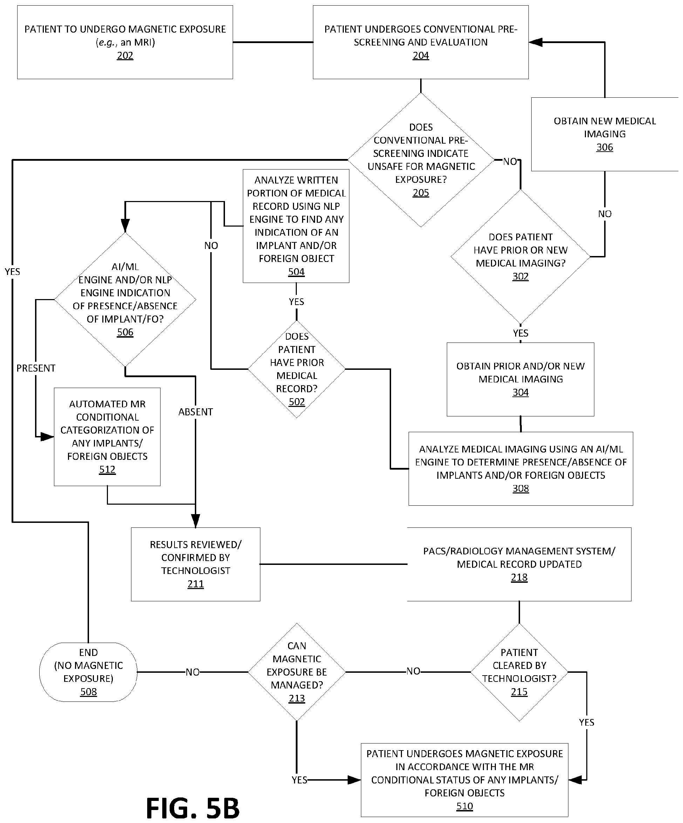

FIG. 5B is an illustration of an improved method of screening for implants and/or foreign objects in a patient's body prior to undergoing magnetic exposure (e.g., an MRI), including determining the availability of any prior medical imaging and/or performing new medical imaging, further including analyzing any textual medical records using a NLP engine, and providing automated MR Conditional categorization for the patient of any detected implants and/or foreign objects based on the analysis;

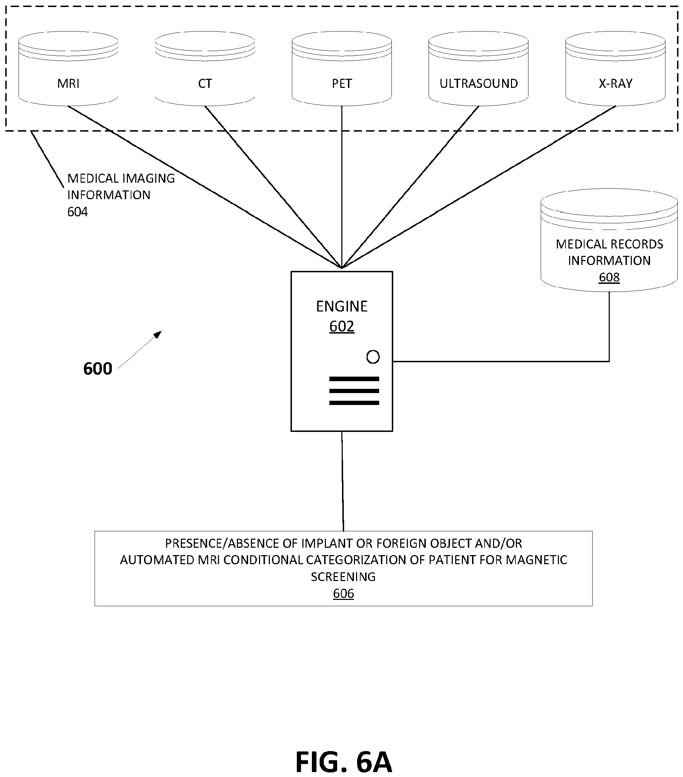

FIG. 6A is an illustration of an exemplary AI/ML engine trained to analyze a variety of medical imaging inputs and/or electronic medical records (EMR), clinical reports, emergency department notes, clinical notes, and the like to determine a presence or absence of an implant or foreign object and/or automated MR Conditional categorization of a patient for magnetic exposure;

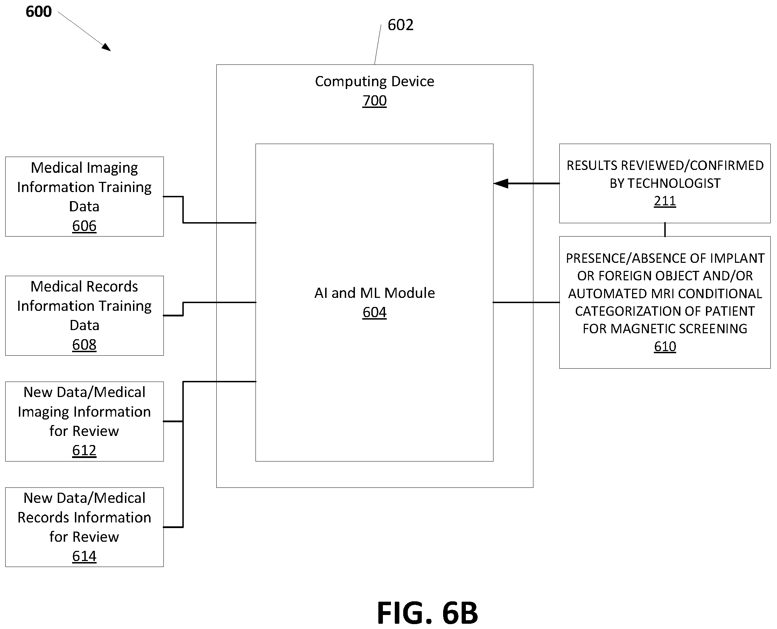

FIG. 6B is an exemplary illustration of training the AI/ML engine to recognize from medical imaging information and/or electronic medical records (EMR), clinical reports, emergency department notes, clinical notes, and the like a presence or absence of implant or foreign object and/or automated MR Conditional categorization of patient for magnetic exposure using images that are known to have/not have implants and/or foreign objects and the conditional categorization of the implants and/or foreign objects; and

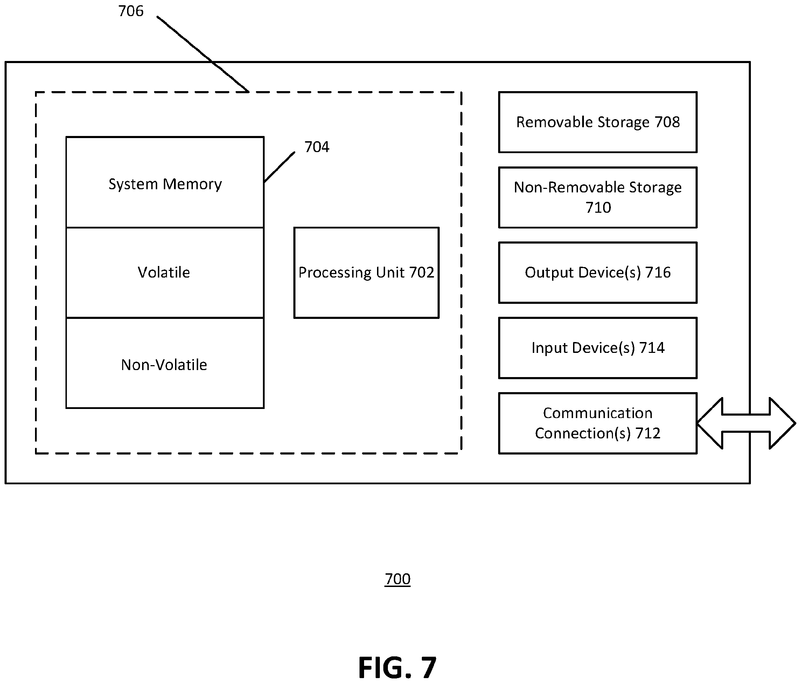

FIG. 7 shows an example computing environment in which example embodiments and aspects may be implemented.

### DETAILED DESCRIPTION

Before the present methods and systems are disclosed and described, it is to be understood that the methods and systems are not limited to specific synthetic methods, specific components, or to particular compositions. It is also to be understood that the terminology used herein is for the purpose of describing particular embodiments only and is not intended to be limiting.

As used in the specification and the appended claims, the singular forms "a," "an" and "the" include plural referents unless the context clearly dictates otherwise. Ranges may be expressed herein as from "about" one particular value, and/or to "about" another particular value. When such a range is expressed, another embodiment includes, from the one particular value and/or to the other particular value. Similarly, when values are expressed as approximations, by use of the antecedent "about," it will be understood that the particular value forms another embodiment. It will be further understood that the endpoints of each of the ranges are significant both in relation to the other endpoint, and independently of the other endpoint.

"Optional" or "optionally" means that the subsequently described event or circumstance may or may not occur, and that the description includes instances where said event or circumstance occurs and instances where it does not.

Throughout the description and claims of this specification, the word "comprise" and variations of the word, such as "comprising" and "comprises," means "including but not limited to," and is not intended to exclude, for example, other additives, components, integers or steps. "Exemplary" means "an example of" and is not intended to convey an indication of a preferred or ideal embodiment. "Such as" is not used in a restrictive sense, but for explanatory purposes.

Disclosed are components that can be used to perform the disclosed methods and systems. These and other components are disclosed herein, and it is understood that when combinations, subsets, interactions, groups, etc. of these components are disclosed that while specific reference of each various individual and collective combinations and permutation of these may not be explicitly disclosed, each is specifically contemplated and described herein, for all methods and systems. This applies to all aspects of this application including, but not limited to, steps in disclosed methods. Thus, if there are a variety of additional steps that can be performed it is understood that each of these additional steps can be performed with any specific embodiment or combination of embodiments of the disclosed methods.

The present methods and systems may be understood more readily by reference to the following detailed description of preferred embodiments and to the Figures and their previous and following description.

As will be appreciated by one skilled in the art, the methods and systems may take the form of an entirely hardware embodiment, an entirely software embodiment, or an embodiment combining software and hardware aspects. Furthermore, the methods and systems may take the form of a computer program product on a computer-readable storage medium having computer-readable program instructions (e.g., computer software) embodied in the storage medium. More particularly, the present methods and systems may take the form of web-implemented computer software. Any suitable computer-readable storage medium may be utilized including hard disks, CD-ROMs, optical storage devices, or magnetic storage devices.

Embodiments of the methods and systems are described below with reference to block diagrams and flowchart illustrations of methods, systems, apparatuses and computer program products. It will be understood that each block of the block diagrams and flowchart illustrations, and combinations of blocks in the block diagrams and flowchart illustrations, respectively, can be implemented by computer program instructions. These computer program instructions may be loaded onto a general-purpose computer, special purpose computer, or other programmable data processing apparatus to produce a machine, such that the instructions which execute on the computer or other programmable data processing apparatus create a means for implementing the functions specified in the flowchart block or blocks.

These computer program instructions may also be stored in a computer-readable memory that can direct a computer or other programmable data processing apparatus to function in a particular manner, such that the instructions stored in the computer-readable memory produce an article of manufacture including computer-readable instructions for implementing the function specified in the flowchart block or blocks. The computer program instructions may also be loaded onto a computer or other programmable data processing apparatus to cause a series of operational steps to be performed on the computer or other programmable apparatus to produce a computer-implemented process such that the instructions that execute on the computer or other programmable apparatus provide steps for implementing the functions specified in the flowchart block or blocks.

Accordingly, blocks of the block diagrams and flowchart illustrations support combinations of means for performing the specified functions, combinations of steps for performing the specified functions and program instruction means for performing the specified functions. It will also be understood that each block of the block diagrams and flowchart illustrations, and combinations of blocks in the block diagrams and flowchart illustrations, can be implemented by special purpose hardware-based computer systems, including for example cloud-based systems, that perform the specified functions or steps, or combinations of special purpose hardware and computer instructions.

FIG. 2A is an illustration of an improved method of screening for implants and/or foreign objects in a patient's body prior to undergoing magnetic exposure (e.g., an MRI) utilizing artificial intelligence (Al) and/or machine learning (ML). The process starts at 202 with a patient that is to undergo some form of magnetic exposure. Generally, magnetic exposure as used herein can be equated with an MRI but can include exposure to any high-strength magnetic field. At 204, the patient undergoes conventional screening and evaluation. This may include asking the patient or someone associated with the patient about any implants, foreign objects, and the like. It also may include some form of a written questionnaire.

At 206, prior medical imaging of the patient is obtained. This prior medical imaging information may be obtained from a PACS or radiology management system used to archive the patient's medical history. In many instances, this information is obtained electronically using a network such as the internet. The patient or someone with authorization authorizes the retrieval of the prior medical imaging information in accordance with all applicable laws and guidelines (e.g., HIPAA, in the United States). As used herein, prior medical imaging information is generally described as medical imaging information obtained prior to scheduling the present magnetic screening of the patient. At 208, a trained AI/ML engine is used to analyze the prior medical imaging information for any indication of an implant or foreign object. Training the AI/ML engine is described in further detail herein. At 210, the AI/ML engine provides an indication of a presence/absence of an implant or a foreign object with a confidence level or probability, and/or automated MR Conditional categorization of the patient for magnetic exposure. In some instances, the AI/ML engine provides a probability or at least a likelihood score that may be correlated with the presence/absence of an implant or foreign object. For example, a threshold may be set at a probability or likelihood score of .5, with a probability of 0.5 or above indicating the presence of an implant or foreign object, and a probability or likelihood score of less than 0.5 indicating the absence of an implant or foreign object. It is to be appreciated that this threshold may be adjusted to get better recall or precision. This indication may be provided to, for example, a PACS and/or radiology management system used for the magnetic screening. MR Conditional categorization is also described in greater detail herein.

At 211, the results of the step 210 are confirmed by a technologist. As used herein, "technologist" may mean someone trained and authorized to review medical imaging and/or other medical information and make a determination regarding safety of the patient to undergo magnetic exposure based on their review. This may include a radiologist or other types of accredited/allowed experts. At 218 the PACS/radiology management system is updated with information about the determination by the technologist as performed at 211 (i.e., validated information). In this manner, future use of the medical imaging information (at 206) does not require re-analyzing it with the AI/ML engine (at 208).

FIG. 2B is an illustration of an improved method of screening for implants and/or foreign objects in a patient's body prior to undergoing magnetic exposure (e.g., an MRI), including performing/not performing the magnetic exposure or managing magnetic exposure of the implant and/or foreign object based on the analysis. Based on the patient's screening and evaluation 204, a determination is made at 205 whether the pre-screening and evaluation indicate it is unsafe for the patient to undergo the magnetic exposure. If, at 205, it is determined that it is not unsafe for the patient to undergo the magnetic exposure, then at 206, prior medical imaging of the patient is obtained and is analyzed at 208 using the AI/ML engine. In this embodiment, the process includes 212, where the AI/ML engine provides an indication of the presence or absence of an implant and/or foreign object. Regardless of the outcome of 212, the process goes to 211 where the results of the AI/ML engine are reviewed/confirmed by a technologist and then to 218 where the PACS/radiology management system is updated with information about the determination by the technologist as performed at 211 (i.e., validated information). In this manner, future use of the medical imaging information (at 206) does not require re-analyzing it with the AI/ML engine (at 208).

At 215, the technologist, after the review of the AI/ML engine analysis at 211, provides an indication whether the patient is cleared/not cleared for the magnetic exposure. If, at 215, the indication from the technologist after review of the AI/ML engine analysis is that the patient is not cleared, then the process goes to 213. At 213, it is determined whether the magnetic exposure can be managed (e.g., can the magnetic exposure be isolated to a part of the body that does not have an implant and/or foreign object). Generally, this determination is made by a technologist. If, at 213 the magnetic exposure cannot be managed, then the process goes to 214 and ends (no magnetic exposure for the patient). If, at 213, the magnetic exposure can be managed, then the process goes to 216 where the patient undergoes managed magnetic exposure Returning to 215, if the indication is that the patient is cleared by the technologist, then the process goes to 216 where the magnetic exposure occurs.

FIG. 3A is an illustration of an improved method of screening for implants and/or foreign objects in a patient's body prior to undergoing magnetic exposure (e.g., an MRI), including determining the availability of any prior medical imaging and/or performing new medical imaging, and performing/not performing the magnetic exposure or managing magnetic exposure of the implant and/or foreign object based on the analysis. In this embodiment, a determination is made at 302 whether the patient has any prior or new medical imaging information or if any prior or new medical imaging information is available. If, at 302, it is determined that the patient does have prior medical imaging information, then at 304 the prior medical imaging information is obtained, as described herein. If, at 302, it is determined that the patient does not have prior medical imaging information or any new medical imaging information, or that the prior and/or new medical imaging information is not available, then at 306 new medical imaging information is obtained. This new medical imaging information is obtained without using high-strength magnetic fields. For example, non-magnetic imaging modalities such as x-ray, computed tomography (CT), positron emission tomography (PET), ultrasound, and the like may be used so that there is no risk exposing the patient to a magnetic field. As used herein, new medical imaging information can generally be described as medical imaging information obtained since the present magnetic screening was scheduled or associated with the present magnetic screening. The process then goes to 204, where any obtained new medical information is then reviewed by a technologist in the conventional pre-screening and evaluation step. At 308, the prior medical imaging information and/or the new medical imaging information is analyzed using the trained AI/ML engine to analyze the provided medical imaging information for any indication of an implant or foreign object. In this embodiment, the process includes 310, where the AI/ML engine provides an indication whether the patient is cleared for the magnetic exposure, or not. Regardless of the outcome of 310, the process goes to 211 where the results of the AI/ML engine are reviewed/confirmed by a technologist and then to 218 where the PACS/radiology management system is updated with information about the determination by the technologist as performed at 211 (i.e., validated information). In this manner, future use of the medical imaging information (304, 306) does not require re-analyzing it with the AI/ML engine (at 308).

At 215, the technologist, after the review of the AI/ML engine analysis at 211, provides an indication whether the patient is cleared/not cleared for the magnetic exposure. If, at 215, the indication from the technologist after review of the AI/ML engine analysis is that the patient is not cleared, then the process goes to 213. At 213, it is determined whether the magnetic exposure can be managed (e.g., can the magnetic exposure be isolated to a part of the body that does not have an implant and/or foreign object). Generally, this determination is made by a technologist. If, at 213 the magnetic exposure cannot be managed, then the process goes to 312 and ends (no magnetic exposure for the patient). If, at 213, the magnetic exposure can be managed, then the process goes to 314 where the patient undergoes managed magnetic exposure. Returning to 215, if the indication is that the patient is cleared by the technologist, then the process goes to 314 where the magnetic exposure occurs.

A similar process is described in relation to FIG. 3B. FIG. 3B is an illustration of an improved method of screening for implants and/or foreign objects in a patient's body prior to undergoing magnetic screening (e.g., an MRI), including determining the availability of any prior medical imaging and/or performing new medical imaging, and providing automated MR Conditional categorization for the patient of any detected implants and/or foreign objects based on the analysis. In this instance, rather than the AI/ML engine providing an indication that the patient is either cleared or not cleared for magnetic exposure, at 308 the AI/ML engine analyzes the medical imaging information and at 310 provides an indication of the presence or absence of an implant and/or foreign object, as described herein. If, at 310, the AI/ML engine indicates the presence of an implant and/or foreign object, then at 316 the AI/ML engine assigns an MR Conditional categorization to any implants/foreign objects found in the patient. This MR Conditional categorization may be indicated on a PACS or other radiology management system used for the magnetic screening. If, at 310, the AI/ML engine provides an indication of the absence of an implant and/or foreign object, as described herein, then the process goes to 211.

Regardless of the outcome of steps 316 or 310, the process goes to 211 where the results of the AI/ML engine are reviewed/confirmed by a technologist and then to 218 where the PACS/radiology management system is updated with information about the determination by the technologist as performed at 211 (i.e., validated information regarding MR Conditional status of any implants/foreign objects). In this manner, future use of the medical imaging information (304, 306) does not require re-analyzing it with the AI/ML engine (at 308).

At 215, the technologist, after the review of the AI/ML engine analysis at 211 and the assigned MR Conditional categorization of any implants/foreign objects at 316, provides an indication whether the patient is cleared/not cleared for the magnetic exposure. If, at 215, the indication from the technologist after review of the AI/ML engine analysis is that the patient is not cleared, then the process goes to 213. At 213, it is determined whether the magnetic exposure can be managed (e.g., can the magnetic exposure be isolated to a part of the body that does not have an implant and/or foreign object). Generally, this determination is made by a technologist. If, at 213 the magnetic exposure cannot be managed, then the process goes to 312 and ends (no magnetic exposure for the patient). If, at 213, the magnetic exposure can be managed, then the process goes to 318 where the patient undergoes managed magnetic exposure in accordance with the MR Conditional status of any implants/foreign objects. Returning to 215, if the indication is that the patient is cleared by the technologist, then the process goes to 318 where the magnetic exposure occurs in accordance with the MR Conditional status of any implants/foreign objects.

Referring now to FIG. 4, the process of analyzing the medical imaging information and at 316 assigning an MR Conditional categorization to the patient generally comprises assigning one of eight different MR Conditional categorizations 402-416 to the patient, though fewer or greater numbers of MR Conditional categorizations are considered within the scope of this disclosure.

The terminology of MR Conditional categorization is recognized by the American Society for Testing and Materials (ASTM) international and used by the Food and Drug Administration (FDA). The term MR Conditional means that the item has to be scanned under certain conditions within the MRI environment. Parameters that are included in the environment include static magnetic field strength, spatial gradient, dB/dt time varying magnetic fields, radio frequency fields, and specific absorption rate (SAR). Additional parameters may also include conditions of leads for a neurostimulator system.

There are a plurality (generally, eight) different categories that help to define the specific conditions for each object/implant to be scanned while in the MRI environment. These categories are described below.

Conditional 1 (402) - object/implant is safe due to the magnetic field is determined to be mild or weak to the in vivo forces or counterforces present for the object. For example, there are certain prosthetic heart valve protheses and annuloplasty rings that show small amounts of magnetic field interactions when exposed to the MRI environment during testing. However, these interactions are less than when comparing how the environment put exertion on the implants by the beating heart.

Conditional 2 (404) - These particular "weakly" ferromagnetic coils, filters, stents, clips, cardiac occluders, or other implants become firmly in place into the tissue approximately six weeks after being placed. It is determined it is unlikely that these objects will be moved or displaced by being put into the MRI magnetic field.

Conditional 3 (406) - Certain transdermal patches with metallic backing such as (e.g. Deponit^{™} nitroglycerin transdermal delivery system), nausea patches or other metallic components they are not necessarily attracted to the magnetic field but have been reported to heat up during MR examinations. This can cause the patient discomfort and a burn to the individual wearing a transdermal patch with metallic backing. It is recommended that the patch to be removed prior to the MRI. A new patch will need to be replaced immediately following the completion of the MRI. The MRI exam needs to be done only after a consultation has taken place with the patient's provider who originally prescribed the transdermal medication patch.

Conditional 4 (408) - A halo vest or cervical fixation device may have ferromagnetic component parts; however, the magnetic field interactions have not been determined. There has not been a report of patient injury in association with the presence of these devices in the MR field when testing. Nevertheless, issues are still present about possible heating due to the MRI being completed. The guidelines provided in the Product Insert or Instructions for Use for the given device of either halo vest or cervical fixation device need to be reviewed so that they can be carefully followed for the patient's safety. Halo vest and cervical fixation devices that are made from conducting metals may heat excessively during the MRI examination, resulting in serious patient injury. The manufacturer is to be contacted for more information. Recently, some cervical fixation devices have been evaluated for a 3-Tesla MRI.

Conditional 5 (410) - This object/implant is acceptable for a patient to complete the MRI or if an individual in the MRI environment (example: relative), only if specific guidelines and/or recommendations are followed by contacting the manufacturer and/or reviewing the information of the implant/device that is located on the MRI Safety website. Be sure to refer to the specific criteria for performing the MRI examination by reviewing the information for the object on the manufacturer's website. A consultation of the manufacturer of the device/implant provides for the latest safety information.

Conditional 6 (412) - This implant/device was labeled to be MR Conditional according to the terminology specified in the American Society for Testing and Materials (ATSM) International, Designation: F2503. Standard Practice for Marking Medical Devices and Other Items for Safety in the MRI environment. Non-clinical testing demonstrated that the implant/device is MR Conditional. A patient with this implant/device can be scanned safely immediately after placement under the following conditions: Static magnetic field of 3-Tesla or less; Maximum spatial gradient magnetic field of 720-Gauss/cm (a higher value for the spatial gradient magnetic field may apply if properly calculated). The Maximum MR system reported whole-body-averaged specific absorption rate (SAR) of 2W/kg for 15 minutes of scanning (per pulse sequence). Other precautions for this Conditional 6 (412) include MRI-related heating. In non-clinical testing, the implant/device must have produced a temperature rise of less than or equal to 6.0 degrees C using an MR system reported, whole body averaged specific absorption rate (SAR) of 2-W/kg for 15-minutes (per pulse sequence) of scanning in a 3-Tesla MRI system. Furthermore, MRI image quality may be compromised if the area of interest is in the exact same area or relatively close to the position of the implant/device. In some cases, the artifact size is relative to the size of the implant/device as indicated. The manufacturer of the device/implant should be contacted for further information, as needed.

Conditional 7 (414) - This implant/device is not intended for use during the operation of an MRI system for an MRI examination. This device should not be inside of the bore of the MRI system, exposing this device to the time-varying and RF fields that are activated during the MRI examination. The manufacturer of this implant/device should be contacted for further information.

Conditional 8 (416) - This information pertains to an implant/device that has MRI labeling at 1.5 Tesla and 3-Tesla, only. In some cases, information may pertain to a single or two-overlapped versions of a stent. The implant/device is determined to be MR Conditional according to the terminology specified in the American Society for Testing and Materials (ASTM) International, Designation: F2503. Standard Practice for Marking Medical Devices and Other Items for Safety in the MRI Environment. Required environment includes: static magnetic field of 1.5-Tesla and 3-Tesla, only; maximum spatial gradient magnetic field of 720-Gauss/cm (a higher value for the spatial gradient magnetic field applies if properly calculated); maximum MRI system reported whole-body-averaged specific absorption rate (SAR) of 2W/kg for 15 minutes of scanning per pulse sequence. Other challenges include MRI-related heating during the scan. In non-clinical testing, the implant/device must produce the following temperature rises during MRI performed for 15-minutes (i.e., per pulse sequence) in 1.5 Tesla and 3-Tesla MR systems, using an MRI system reported, whole body averaged SAR of 2-W/kg or less, as follows: highest allowable temperature changes must be less than or equal to 6.0 degrees C at 1.5-T/64-MHz; or less than or equal to 6.0 degrees C at 3-T/128-MHz. As described above, MRI image quality may be compromised if the area of interest is in the exact same area or relatively close to the position of the implant/device. In some cases, the artifact size is relative to the size of the implant or device as indicated.

Other categories are MR Unsafe 418 and MR Safe 420. These are not conditional categories. Terminology from the American Society for Testing (ASTM) International and utilized by the Food and Drug Administration refers to MR Unsafe as an item that is known to pose hazards in all MRI environments. MR Unsafe items include magnetic items such as a pair of ferromagnetic scissors. When categorized as MRI Unsafe 1, this means the object is considered to pose a potential or realistic risk or hazard to a patient or individual in the MR environment primarily as the result of movement or displacement of the object/device. Other risks or a different hazard could also exist. Therefore, in general, the presence of this object is a contraindication for an MR examination and/or for an individual to enter the MR environment depending on the nature of the object or item. The "default" static magnetic field strength for an unsafe implant/device is typically 1.5-Tesla.

MRI Unsafe 2 means that the object displays only minor magnetic field interactions which, in consideration of the in vivo application of this object/device, is unlikely to pose a hazard or risk in association with movement or displacement. The presence of this object is considered to be a contraindication for an MR examination or for an individual in the MR environment. Potential risks of performing an MR examination with a patient or individual with this object/device are related to possible induced currents, excessive heating, or other potentially hazardous conditions. Therefore, it is inadvisable to perform MR examination in a patient or individual with the device/object. For example, the Swan-Ganz thermodilution catheter (and other similar catheters) displays no attraction to the MR system. However, there has been a report of a Swan-Ganz catheter that "melted" in a patient during an MR examination. Therefore, the presence of this cardiovascular catheter and any other similar device is considered to be a contraindication for a patient undergoing an MR examination.

MR Safe means that the object is considered to be safe for the patient undergoing an MR procedure or an individual in the MR environment, with special reference to the highest static magnetic field strength that was used for the MR safety test. The object has undergone testing to demonstrate that it is safe, or it is made from material(s) considered to be safe with regard to the MR environment (e.g., plastic, silicone, glass, etc.) or an MR procedure.

Terminology from the American Society for Testing and Materials (ASTM) International and utilized by the Food and Drug Administration refers to MR Safe as an item that poses no known hazards in all MRI environments. Using the current terminology, MR safe items include non-conducting, non-metallic, non-magnetic items such as a plastic Petri dish.

FIG. 4 indicates only an example of MR Conditional categorization. More, fewer or different categorizations are considered within the scope of this disclosure.

FIG. 5A is an illustration of an improved method of screening for implants and/or foreign objects in a patient's body prior to undergoing magnetic exposure (e.g., an MRI), including determining the availability of any prior medical imaging and/or performing new medical imaging, further including analyzing any textual medical records using an NLP engine, and performing/not performing the magnetic exposure or managing magnetic exposure of the implant and/or foreign object based on the analysis. In this instance, after the medical imaging information is analyzed by the trained AI/ML engine at 308, it is determined at 502 whether the patient has any prior medical record information. As used herein, medical records information includes electronic medical records (EMR), clinical reports, emergency department notes, clinical notes, and the like. Generally, this information can include medical records information obtained prior to scheduling the present magnetic screening and/or medical records information obtained in association with the present magnetic screening. If, at 502, it is determined that the patient does have prior medical record information, then at 504 the prior medical information is accessed in accordance with all applicable laws and guidelines (for example, HIPAA, etc.) and is analyzed using a trained NLP engine to identify implanted devices and/or known embedded foreign objects associated with the patient. The process then goes to 506 where an indication is provided based on the analysis of the medical imaging information by the AI/ML engine and the analysis of the medical record by the NLP engine of the presence or absence of an implant and/or foreign object. Returning to 502, if it is determined that the patient does not have prior medical record information or such information is not accessible, then step 504 is skipped and the process goes to 506.

Regardless of the outcome of 506, the process goes to 211 where the results of the AI/ML engine and/or medical record review 504 are reviewed/confirmed by a technologist and then to 218 where the PACS/radiology management system/medical record is updated with information about the determination by the technologist as performed at 211 (i.e., validated information regarding any implants/foreign objects). In this manner, future use of the medical imaging information (304, 306) and/or medical record information does not require re-analyzing it with the AI/ML engine (at 308) and/or NLP engine (at 504).

At 215, the technologist, after the review of the AI/ML engine analysis at 211 and/or NLP engine review of the medical record at 504, provides an indication whether the patient is cleared/not cleared for the magnetic exposure. If, at 215, the indication from the technologist after review of the AI/ML engine analysis and/or NLP engine review of the medical record is that the patient is not cleared, then the process goes to 213. At 213, it is determined whether the magnetic exposure can be managed (e.g., can the magnetic exposure be isolated to a part of the body that does not have an implant and/or foreign object). Generally, this determination is made by a technologist. If, at 213 the magnetic exposure cannot be managed, then the process goes to 508 and ends (no magnetic exposure for the patient). If, at 213, the magnetic exposure can be managed, then the process goes to 510 where the patient undergoes managed magnetic exposure. Returning to 215, if the indication is that the patient is cleared by the technologist, then the process goes to 510 where the magnetic exposure occurs.

FIG. 5B is an illustration of an improved method of screening for implants and/or foreign objects in a patient's body prior to undergoing magnetic exposure (e.g., an MRI), including determining the availability of any prior medical imaging and/or performing new medical imaging, further including analyzing any textual medical records using an NLP engine, and providing automated MR Conditional categorization for the patient of any detected implants and/or foreign objects based on the analysis. In this instance, if at 506 the AI/ML engine and/or the NLP engine provide an indication of the presence of an implant and/or foreign object based on the analysis of the medical imaging information and/or the prior medical records information, and at 512 the AI/ML engine and/or the NLP engine assign an MR Conditional categorization to any implants and/or foreign objects found within the patient (described herein). This assigned MR Conditional categorization may be indicated on a PACS or other radiology management system used for the magnetic screening. If, at 506, the AI/ML engine and/or the NLP engine provide an indication of the absence of an implant and/or foreign object, then the process skips 512 and goes to 211.

In this instance, after the medical imaging information is analyzed by the trained AI/ML engine at 308, it is determined at 502 whether the patient has any prior medical record information. If, at 502, it is determined that the patient does have prior medical record information, then at 504 the prior medical information is analyzed using a trained NLP engine to identify implanted devices and/or known embedded foreign objects associated with the patient. The process then goes to 512 where it is determined based on the analysis of the medical imaging information by the AI/ML engine and the analysis of the medical record by the NLP engine whether the patient is cleared for magnetic exposure. Returning to 502, if it is determined that the patient does not have prior medical record information or such information is not accessible, then step 504 is skipped and the process goes directly to 512.

Regardless of the outcome of 512, the process goes to 211 where the results of the AI/ML engine and/or medical record review 504 are reviewed/confirmed by a technologist and then to 218 where the PACS/radiology management system/medical record is updated with information about the determination by the technologist as performed at 211 (i.e., validated information regarding any implants/foreign objects). In this manner, future use of the medical imaging information (304, 306) and/or medical record information does not require re-analyzing it with the AI/ML engine (at 308) and/or NLP engine (at 504).

At 215, the technologist, after the review of the AI/ML engine analysis at 211 and/or NLP engine review of the medical record at 504, provides an indication whether the patient is cleared/not cleared for the magnetic exposure. If, at 215, the indication from the technologist after review of the AI/ML engine analysis and/or NLP engine review of the medical record is that the patient is not cleared, then the process goes to 213. At 213, it is determined whether the magnetic exposure can be managed (e.g., can the magnetic exposure be isolated to a part of the body that does not have an implant and/or foreign object). Generally, this determination is made by a technologist. If, at 213 the magnetic exposure cannot be managed, then the process goes to 508 and ends (no magnetic exposure for the patient). If, at 213, the magnetic exposure can be managed, then the process goes to 510 where the patient undergoes managed magnetic exposure. Returning to 215, if the indication is that the patient is cleared by the technologist, then the process goes to 510 where the magnetic exposure occurs.

It is to be appreciated that while FIGS. 5A and 5B show analysis of the prior medical imaging information 308 using the AI/ML engine before analysis 504 of the patient's prior medical record using the NLP engine, this is for illustrative purposes only and the methods are not limited to this order of events. In some instances, the NLP analysis 504 of the patient's prior medical record can be performed prior to the analysis of the prior medical imaging information 308 using the AI/ML engine. In some instances, analysis 504 of the patient's prior medical record using the NLP engine can be used in lieu of the analysis of the prior medical imaging information 308 to make a determination about the patient having an implant and/or foreign object instead of performing both types of analyses.

FIG. 6A is an illustration of an exemplary AI/ML engine and/or NLP trained to analyze a variety of medical imaging input and determine a presence or absence of an implant or foreign object and/or automated MR Conditional categorization of a patient for magnetic exposure.

As shown in FIG. 6A, the system 600 comprises a computer-implemented artificial intelligence-enabled engine 602. The term "artificial intelligence" is defined herein to include the capability of a functional unit to perform functions that are generally associated with human intelligence such as reasoning and learning.. Al includes, but is not limited to, knowledge bases, machine-learning, representation learning, and deep learning. The term "machine-learning" is defined herein to be a subset of Al that enables a machine to acquire knowledge by extracting patterns from raw data. Machine-learning techniques include, but are not limited to, logistic regression, support vector machines (SVMs), decision trees (including randomized decision forests), Naïve Bayes classifiers, AutoRegressive Integrated Moving Average (ARIMA) machine-learning algorithms, and artificial neural networks. The term "representation learning" is defined herein to be a subset of machine-learning that enables a machine to automatically discover representations needed for feature detection, prediction, or classification from raw data. Representation learning techniques include, but are not limited to, autoencoders. The term "deep learning" is defined herein to be a subset of machine-learning that that enables a machine to automatically discover representations needed for feature detection, prediction, classification, etc. using layers of processing. Deep learning techniques include, but are not limited to, artificial neural network (including deep nets, long short-term memory (LSTM) recurrent neural network (RNN) architecture), or multilayer perceptron (MLP). Machine-learning models include supervised, semi-supervised, and unsupervised learning models. In a supervised learning model, the model learns a function that maps an input (also known as feature or features) to an output (also known as a target or target) during training with a labeled data set (or dataset). In an unsupervised learning model, the model learns a function that maps an input (also known as feature or features) to an output during training with an unlabeled data set. In a semi-supervised model, the model learns a function that maps an input (also known as feature or features) to an output (also known as a target or target) during training with both labeled and unlabeled data. In some instances, the Al may comprise natural language processing (NLP). NLP refers to a branch of Al concerned with giving computers the ability to understand text and spoken words in much the same way human beings can.

The engine 602 comprises AI that has been trained to analyze medical imaging information 604 and/or medical record information 608 and predict the presence or absence of an implant or a foreign object in the patient with a confidence level or probability, and/or predict an MR Conditional categorization of patient for magnetic exposure 606. This may comprise providing a binary indication of cleared/not cleared for magnetic exposure for the patient, or provide an MR Conditional categorization for the patient that can be used by a healthcare worker to assess risks of exposing the patient to a magnetic field and/or manage the patient's exposure. Non-limiting examples of medical imaging information include MRIs, CTs, PET scans, ultrasounds, x-rays, and the like. This information is used by the healthcare worker cooperative with conventional pre-screening and evaluation information about the patient to make a determination about conducting or managing magnetic exposure of the patient.

Optionally or alternatively, in some instances the engine 602 may comprise Al that has been trained to analyze medical record information 608 and predict the presence or absence of an implant or a foreign object in the patient with a confidence level or probability, and/or predict an MR Conditional categorization of patient for magnetic exposure 606. This NLP analysis may be performed sequentially or in parallel with, or independently of the analysis of the medical imaging information 604 by the engine. It is to be appreciated that the engine 602 may not comprise a singular computing device but may be comprised of a plurality of computing devices at one or disparate locations in communication with one another. It is also to be appreciate that the same computing device may be used as an AI/ML engine and a NLP engine, or each engine may be associated with a separate computing device.

As shown in FIG. 6B, the engine 602 includes an AI/ML machine-learning (e.g., training) module 604 trained for processing new data on which to predict a condition. Training the AI/ML module 604 uses training data 606, 608, which may comprise information associated with a stimulus that may indicate a condition. The training data may comprise information associated with known implants or foreign objects visible in medical imaging information and/or medical imaging information that is known to not show an implant or foreign object 6064. In other instances, the training data may comprise information associated with known implants or foreign objects described in medical records information and/or medical records information that is known to not describe an implant or foreign object 606. Generally, the training data 606, 608 is comprised of historical data extracted from prior medical imaging information and/or medical records information. Furthermore, as the disclosed processes utilize human validation 211 of the predicted condition 610, these results may be used for continuous learning of the AI/ML module 604. The AI/ML module 604 is further configured to identify the individual independent variables that are used by the trained Al to make predictions, which may be considered a dependent variable. For example, the training data 606, 608 may be generally unprocessed or formatted and include extra information in addition to medical imaging information and/or medical records information. For example, the medical records information may include account codes, codes associated with the services performed by the provider, address information, and the like, which can be filtered out by the AI/ML module 604. The features extracted from the training data 606, 608 may be called attributes and the number of features may be called the dimension. The AI/ML module 604 may further be configured to assign defined labels to the training data 606, 608 and to the generated predictions to ensure a consistent naming convention for both the input features and the predicted conditions. The AI/ML module 604 processes both the featured training data 606, 608 including the labels, and may be configured to test numerous functions to establish a quantitative relationship between the featured and labeled input data and the predicted outputs. The AI/ML module 604 may use modeling techniques, as described herein, to evaluate the effects of various input data features on the predicted outputs. These effects may then be used to tune and refine the quantitative relationship between the featured and labeled input data and the predicted outputs. The tuned and refined quantitative relationship between the featured and labeled input data generated by the AI/ML module 604 is output for use in the trained AI. The machine-learning used by the AI/ML module 604 may be referred to as a machine-learning algorithm.

The trained Al of the AI/ML module 604 is used for processing new data 612, 614 on which to make condition predictions 610 using the new data based on training. The new data 612, 614 may be the same data/information as the training data 606, 608 in content and form except the new data will be used for an actual event forecast or condition prediction. The trained AI/ML module 604 may, in effect, be generated by the AI/ML module 604 in the form of the quantitative relationship determined between the featured and labeled input data and the predicted outputs. The trained AI/ML module 604 may, in some embodiments, be referred to as an Al model. The trained AI/ML module 604 may be configured to output predicted conditions 610, as described herein, including the presence or absence of an implant or a foreign object with a confidence level or probability, and/or an automated MR Conditional categorization of a patient for magnetic exposure 610.

The trained AI/ML module 604 may be configured to communicate the event prediction 606 in a variety of formats and may include additional information, including, but not limited to, illustrations of the event in comparisons to an idealized version of the event, comparison of the event outcome relative to one or more of the featured inputs, and trends in the event outcomes including a breakdown of such trends relative to one or more of the featured inputs. In some embodiments, the predicted conditions 610 are generated based on stimulus, such as medical imaging information and/or medical records information. The trained AI/ML module 604 may be continually or periodically re-trained as new data 606, 608, including validation 211 of its own condition predictions 610 is received by the AI/ML module 604.

FIG. 7 shows an example computing environment in which example embodiments and aspects may be implemented. The computing device environment is only one example of a suitable computing environment and is not intended to suggest any limitation as to the scope of use or functionality.

Numerous other general purpose or special purpose computing devices environments or configurations may be used. Examples of well-known computing devices, environments, and/or configurations that may be suitable for use include, but are not limited to, personal computers, server computers, handheld or laptop devices, multiprocessor systems, cloud-based systems, microprocessor-based systems, network personal computers (PCs), minicomputers, mainframe computers, embedded systems, distributed computing environments that include any of the above systems or devices, and the like. The computing environment may include a cloud-based computing environment.

Computer-executable instructions, such as program modules, being executed by a computer may be used. Generally, program modules include routines, programs, objects, components, data structures, etc. that perform particular tasks or implement particular abstract data types. Distributed computing environments may be used where tasks are performed by remote processing devices that are linked through a communications network or other data transmission medium. In a distributed computing environment, program modules and other data may be located in both local and remote computer storage media including memory storage devices.

With reference to FIG. 7, an example system for implementing aspects described herein includes a computing device, such as computing device 700. In its most basic configuration, computing device 700 typically includes at least one processing unit 702 and memory 704. Depending on the exact configuration and type of computing device, memory 704 may be volatile (such as random-access memory (RAM)), non-volatile (such as read-only memory (ROM), flash memory, etc.), or some combination of the two. This most basic configuration is illustrated in FIG. 7 by dashed line 706.

Computing device 700 may have additional features/functionality. For example, computing device 700 may include additional storage (removable and/or non-removable) including, but not limited to, magnetic or optical disks or tape. Such additional storage is illustrated in FIG. 7 by removable storage 708 and non-removable storage 710.

Computing device 700 typically includes a variety of computer readable media. Computer readable media can be any available media that can be accessed by the device 700 and includes both volatile and non-volatile media, removable and non-removable media.

Computer storage media include volatile and non-volatile, and removable and non-removable media implemented in any method or technology for storage of information such as computer readable instructions, data structures, program modules or other data. Memory 704, removable storage 708, and non-removable storage 710 are all examples of computer storage media. Computer storage media include, but are not limited to, RAM, ROM, electrically erasable program read-only memory (EEPROM), flash memory or other memory technology, CD-ROM, digital versatile disks (DVD) or other optical storage, magnetic cassettes, magnetic tape, magnetic disk storage or other magnetic storage devices, or any other medium which can be used to store the desired information, and which can be accessed by computing device 700. Any such computer storage media may be part of computing device 700.

Computing device 700 may contain communication connection(s) 712 that allow the device to communicate with other devices. Computing device 700 may also have input device(s) 714 such as a keyboard, mouse, pen, voice input device, touch input device, etc. Output device(s) 716 such as a display, speakers, printer, etc. may also be included. All these devices are well known in the art and need not be discussed at length here.

The computing system 700 described herein may comprise all or part of an artificial neural network (ANN) or a convolutional neural network (CNN). An ANN is a computing system including a plurality of interconnected neurons (e.g., also referred to as "nodes"). This disclosure contemplates that the nodes can be implemented using a computing device (e.g., a processing unit and memory as described herein), such as computing device 700 described herein. The nodes can be arranged in a plurality of layers such as input layer, output layer, and optionally one or more hidden layers. An ANN having hidden layers can be referred to as deep neural network or multilayer perceptron (MLP). Each node is connected to one or more other nodes in the ANN. For example, each layer is made of a plurality of nodes, where each node is connected to all nodes in the previous layer. The nodes in a given layer are not interconnected with one another, i.e., the nodes in a given layer function independently of one another. As used herein, nodes in the input layer receive data from outside of the ANN, nodes in the hidden layer(s) modify the data between the input and output layers, and nodes in the output layer provide the results. Each node is configured to receive an input, implement an activation function (e.g., binary step, linear, sigmoid, tanH, or rectified linear unit (ReLU) function), and provide an output in accordance with the activation function. Additionally, each node is associated with a respective weight. ANNs are trained with a dataset to maximize or minimize an objective function (e.g., the business goals and objectives). In some implementations, the objective function is a cost function, which is a measure of the ANN's performance (e.g., error such as L1 or L2 loss) during training, and the training algorithm tunes the node weights and/or bias to minimize the cost function. This disclosure contemplates that any algorithm that finds the maximum or minimum of the objective function can be used for training the ANN. Training algorithms for ANNs include, but are not limited to, backpropagation. It should be understood that an artificial neural network is provided only as an example machine-learning model. This disclosure contemplates that the machine-learning model can be any supervised learning model, semi-supervised learning model, or unsupervised learning model. Optionally, the machine-learning model is a deep learning model. Machine-learning models are known in the art and are therefore not described in further detail herein.

A convolutional neural network (CNN) is a type of deep neural network that can be applied, for example, to non-linear workflow prediction applications, such as those described herein. Unlike traditional neural networks, each layer in a CNN has a plurality of nodes arranged in three dimensions (width, height, depth). CNNs can include different types of layers, e.g., convolutional, pooling, and fully-connected (also referred to herein as "dense") layers. A convolutional layer includes a set of filters and performs the bulk of the computations. A pooling layer is optionally inserted between convolutional layers to reduce the computational power and/or control overfitting (e.g., by downsampling). A fully-connected layer includes neurons, where each neuron is connected to all of the neurons in the previous layer. The layers are stacked similar to traditional neural networks. GCNNs are CNNs that have been adapted to work on structured datasets such as graphs.

Other supervised learning models that may be utilized according to embodiments described herein include a logistic regression (LR) classifier, a Naïve Bayes' (NB) classifier, a k-NN classifier, a majority voting ensemble, and the like.

A LR classifier is a supervised classification model that uses the logistic function to predict the probability of a target, which can be used for classification. LR classifiers are trained with a data set (also referred to herein as a "dataset") to maximize or minimize an objective function, for example a measure of the LR classifier's performance (e.g., error such as L1 or L2 loss), during training. This disclosure contemplates that any algorithm that finds the minimum of the cost function can be used. LR classifiers are known in the art and are therefore not described in further detail herein.

A NB classifier is a supervised classification model that is based on Bayes' Theorem, which assumes independence among features (i.e., presence of one feature in a class is unrelated to presence of any other features). NB classifiers are trained with a data set by computing the conditional probability distribution of each feature given label and applying Bayes' Theorem to compute conditional probability distribution of a label given an observation. NB classifiers are known in the art and are therefore not described in further detail herein.

A k-NN classifier is a supervised classification model that classifies new data points based on similarity measures (e.g., distance functions). k-NN classifiers are trained with a data set (also referred to herein as a "dataset") to maximize or minimize an objective function, for example a measure of the k-NN classifier's performance, during training. This disclosure contemplates that any algorithm that finds the maximum or minimum of the objective function can be used. k-NN classifiers are known in the art and are therefore not described in further detail herein.

A majority voting ensemble is a meta-classifier that combines a plurality of machine-learning classifiers for classification via majority voting. In other words, the majority voting ensemble's final prediction (e.g., class label) is the one predicted most frequently by the member classification models. Majority voting ensembles are known in the art and are therefore not described in further detail herein.

It should be understood that the various techniques described herein may be implemented in connection with hardware components or software components or, where appropriate, with a combination of both. Illustrative types of hardware components that can be used include Field-programmable Gate Arrays (FPGAs), Application-specific Integrated Circuits (ASICs), Application-specific Standard Products (ASSPs), System-on-a-chip systems (SOCs), Complex Programmable Logic Devices (CPLDs), etc. The methods and apparatus of the presently disclosed subject matter, or certain aspects or portions thereof, may take the form of program code (i.e., instructions) embodied in tangible media, such as floppy diskettes, CD-ROMs, hard drives, or any other machine-readable storage medium where, when the program code is loaded into and executed by a machine, such as a computer, the machine becomes an apparatus for practicing the presently disclosed subject matter.

Although exemplary implementations may refer to utilizing aspects of the presently disclosed subject matter in the context of one or more stand-alone computer systems, the subject matter is not so limited, but rather may be implemented in connection with any computing environment, such as a network or distributed computing environment. Still further, aspects of the presently disclosed subject matter may be implemented in or across a plurality of processing chips or devices, and storage may similarly be effected across a plurality of devices. Such devices might include personal computers, network servers, and handheld devices, for example.

Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims.

## Claims

1. A system for screening for implants and/or foreign objects prior to undergoing magnetic exposure, the system comprising:
at least one computing device, wherein the at least one computing device comprises an artificial intelligence (AI) trained module, said AI trained module trained using historical medical information; and
a memory storing computer-readable instructions that when executed by the at least one computing device cause the at least one computing device to:
receive, by the AI trained module, medical information about a patient;
analyze, by the AI trained module, the received medical information; and
provide, by the AI trained module, information related to performing the magnetic exposure of the patient based on the analysis of the received medical information.

2. The system of claim 1, wherein the medical information about the patient comprises medical imaging information.

3. The system of claim 2, wherein providing the information related to performing the magnetic screening on the patient based on the analysis of the received medical information comprises the trained AI module analyzing the medical imaging information and indicating a presence or an absence of an implant or a foreign object in the patient's body with a confidence level or probability, and/or indicating an MR Conditional categorization for the implant or the foreign object in the patient's body.

4. The system of claim 1, wherein the medical information about the patient comprises medical records information.

5. The system of claim 4, wherein providing the information related to performing the magnetic exposure of the patient based on the analysis of the received medical information comprises the trained AI module analyzing the medical records information and indicating a presence or an absence of an implant or a foreign object in the patient's body with a confidence level or probability, and/or indicating an MR Conditional categorization for the implant or the foreign object in the patient's body.

6. The system of claim 1, wherein the medical information about the patient comprises medical imaging information and medical records information.

7. The system of claim 6, wherein providing the information related to performing the magnetic screening on the patient based on the analysis of the received medical information comprises the trained AI module analyzing the medical records information and the medical imaging information and indicating a presence or an absence of an implant or a foreign object in the patient's body with a confidence level or probability, and/or indicating an MR Conditional categorization for the implant or the foreign object in the patient's body.

8. The system of any of claims 1-7, wherein the information related to performing the magnetic exposure of the patient based on the analysis of the received medical information provided by the trained AI module is verified by a technologist.

9. A method for screening for implants and/or foreign objects prior to undergoing magnetic exposure, comprising:
training an artificial-intelligence (AI) module using historical medical records;
receiving, by the artificial-intelligence (AI) trained module, medical information about a patient;
analyzing, by the AI trained module, the received medical information; and
providing, by the AI trained module, information related to performing the magnetic exposure of the patient based on the analysis of the received medical information.

10. The method of claim 9, wherein the medical information about the patient comprises medical imaging information.

11. The method of claim 10, wherein providing the information related to performing the magnetic exposure of the patient based on the analysis of the received medical information comprises the trained AI module analyzing the medical imaging information and indicating a presence or an absence of an implant or a foreign object in the patient's body with a confidence level or probability, and/or indicating an MR Conditional categorization for the implant or the foreign object in the patient's body.

12. The method of claim 9, wherein the medical information about the patient comprises medical records information.

13. The method of claim 12, wherein providing the information related to performing the magnetic exposure of the patient based on the analysis of the received medical information comprises the trained AI module analyzing the medical records information and indicating a presence or an absence of an implant or a foreign object in the patient's body with a confidence level or probability, and/or indicating an MR Conditional categorization for the implant or the foreign object in the patient's body.

14. The method of claim 9, wherein the medical information about the patient comprises medical imaging information and medical records information.

15. The method of claim 14, wherein providing the information related to performing the magnetic screening on the patient based on the analysis of the received medical information comprises the trained AI module analyzing the medical records information and the medical imaging information and indicating a presence or an absence of an implant or a foreign object in the patient's body with a confidence level or probability, and/or indicating an MR Conditional categorization for the implant or the foreign object in the patient's body.
